(19) Europäisches Patentamt
European Patent Office
Office européen des brevets

(11) Veröffentlichungsnummer: **0 129 798**
**A1**

(12) # EUROPÄISCHE PATENTANMELDUNG

(21) Anmeldenummer: **84106861.2**

(22) Anmeldetag: **15.06.84**

(51) Int. Cl.[4]: **C 07 D 249/08**
C 07 D 231/12, C 07 D 233/60
C 07 D 235/06, A 01 N 43/64
A 01 N 43/50, A 01 N 43/52
A 01 N 43/56

(30) Priorität: **23.06.83 DE 3322526**

(43) Veröffentlichungstag der Anmeldung:
**02.01.85 Patentblatt 85/1**

(84) Benannte Vertragsstaaten:
**AT BE CH DE FR GB IT LI NL SE**

(71) Anmelder: **BASF Aktiengesellschaft**
**Carl-Bosch-Strasse 38**
**D-6700 Ludwigshafen(DE)**

(72) Erfinder: **Roeser, Karl, Dr.**
**Muldweg 11**
**D-6945 Hirschberg(DE)**

(72) Erfinder: **Buschmann, Ernst, Dr.**
**Georg-Ludwig-Krebs-Strasse 10**
**D-6700 Ludwigshafen(DE)**

(72) Erfinder: **Ammermann, Eberhard, Dr.**
**Sachsenstrasse 3**
**D-6700 Ludwigshafen(DE)**

(72) Erfinder: **Pommer, Ernst-Heinrich, Dr.**
**Berliner Platz 7**
**D-6703 Limburgerhof(DE)**

(54) **Fungizide Azolverbindungen, ihre Herstellung und Verwendung.**

(57) Azolverbindung der Formel I,

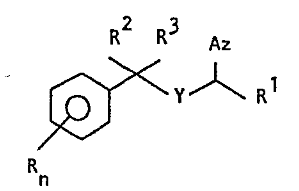

I,

in welcher

Rn bis zu 5 gleiche oder verschiedene Substituenten bedeutet, ausgewählt aus der Gruppe Halogen, Alkyl, Alkoxy, Alkylthio, Alkylsulfonyl, Halogenalkyl, Nitro, Cyano, ggf. substituiertes Phenyl, ggf. substituiertes Phenoxy oder Wasserstoff,

R¹ einen ggf. verzweigten Alkylrest, einen Cycloalkylrest, einen Cycloalkylalkylrest, einen ggf. substituierten Arylrest, einen ggf. substituierten Aralkylrest, einen ggf. substituierten Aryloxyalkyrest, einen ggf. substituierten Benzyloxyalkylrest, einen ggf. substituierten Alkenylrest oder einen ggf. substituierten Alkinylrest bedeutet,

R² und R³ unabhängig voneinander Alkyl oder gemeinsam ein Diradikal der Formel $-(CH_2)_m-$ bedeuten, wobei m für eine ganze Zahl von 2 bis 7 steht,

Az 1-(1,2,4-Triazolyl), 4-(1,2,4-Triazolyl), 1-Imidazolyl, 1-Pyrazolyl oder 1-Benzimidazolyl bedeutet,

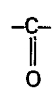
oder
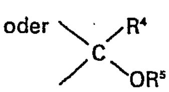
bedeutet, wobei

R⁴ Wasserstoff, $C_1$- bis $C_4$-Alkyl, Vinyl oder Allyl und

R⁵ Wasserstoff, $C_1$- bis $C_3$-Alkyl, ggf. subst. Alkenyl, ggf. subst.

Alkinyl oder ggf. subst. Benzyl bedeutet,

die biologisch annehmbaren Salze oder Komplexverbindungen der Verbindungen der Formel I, ein Verfahren und ein spezielles Verfahren zur Herstellung von Verbindungen der Formel I und deren Verwendung in fungiziden Pflanzenschutzmitteln.

EP 0 129 798 A1

Fungizide Azolverbindungen, ihre Herstellung und Verwendung

Es ist bekannt, Azolverbindungen als Fungizide zu verwenden (DE-OS 30 48 266, EPA 55 833).

Es ist eine Aufgabe der Erfindung, neue Azolverbindungen anzugeben, die in ihrer Wirksamkeit, insbesondere bei niedrigen Konzentrationen, den bekannten Verbindungen entweder überlegen sind oder/und beim Auftreten von Resistenzerscheinungen alternativ angewendet werden können.

Es wurden neue Azolylverbindungen der Formel I gefunden

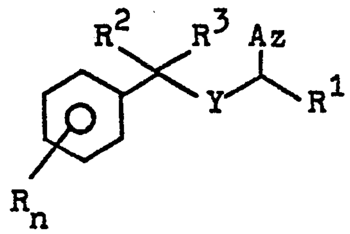 I,

in welcher

$R_n$ bis zu 5 gleiche oder verschiedene Substituenten bedeutet, ausgewählt aus der Gruppe Halogen, Alkyl, Alkoxy, Alkylthio, Alkylsulfonyl, Halogenalkyl, Nitro, Cyano, ggf. substituiertes Phenyl, ggf. substituiertes Phenoxy oder Wasserstoff,

$R^1$ einen ggf. verzweigten Alkylrest, einen Cycloalkylrest, einen Cycloalkylalkylrest, einen ggf. substituierten Arylrest, einen ggf. substituierten Aralkylrest, einen ggf. substituierten Aryloxyalkylrest, einen ggf. substituierten Benzyloxyalkylrest, einen ggf. substituierten Alkenylrest oder einen ggf. substituierten Alkinylrest bedeutet,

$R^2$ und $R^3$ unabhängig voneinander Alkyl oder gemeinsam ein Diradikal der Formel $-(CH_2)_m-$ bedeuten, wobei m für eine ganze Zahl von 2 bis 7 steht,

Az 1-(1,2,4-Triazolyl), 4-(1,2,4-Triazolyl), 1-Imidazolyl, 1-Pyrazolyl oder 1-Benzimidazolyl bedeutet,

Y 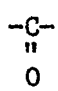 oder 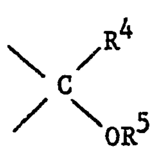

bedeutet, wobei

Mu/P

$R^4$ Wasserstoff, $C_1$- bis $C_4$-Alkyl, Vinyl oder Allyl und

$R^5$ Wasserstoff, $C_1$- bis $C_3$-Alkyl, ggf. subst. Alkenyl, ggf. subst. Alkinyl oder ggf. subst. Benzyl bedeutet.

Diese Verbindungen sowie deren pflanzenverträgliche Metallkomplexe und Säureadditionssalze haben meistens eine bessere fungizide Wirkung als die bekannten Fungizide. Mindestens eignen sie sich als gleichwertiger Ersatz bekannter Fungizide zur Bekämpfung resistent gewordener Stämme von Phytopathogenen.

In der Formel I bedeuten beispielsweise:
R Halogen (Fluor, Chlor, Brom oder Iod), Nitro, Cyano, Alkyl mit 1 bis 4 Kohlenstoffatomen wie z.B. Methyl, Ethyl, n-Propyl, iso-Propyl, Cyclopropyl, n-Butyl, iso-Butyl, sec.-Butyl, tert.-Butyl oder Cyclobutyl, Alkylsulfonyl wie z.B. Methylsulfonyl, Ethylsulfonyl, n-Propylsulfonyl oder n-Butylsulfonyl, Alkoxy mit 1 bis 2 Kohlenstoffatomen, Alkylthio mit 1 bis 2 Kohlenstoffatomen, Halogenalkyl mit 1 bis 4 Kohlenstoffatomen und 1 bis 5 Halogenatomen, insbesondere mit 1 bis 2 Kohlenstoffatomen und 1 bis 3 gleichen oder verschiedenen Halogenatomen, wobei als Halogenatome vorzugsweise Fluor oder Chlor geeignet sind, und als Beispiel für Halogenalkyl Trifluormethyl genannt wird.

R bedeutet vorzugsweise gegebenenfalls einfach oder mehrfach (zwei- bis dreifach), gleichartig oder verschieden substituiertes Phenyl oder Phenoxy, wobei jeweils als Substituenten bevorzugt sind: Halogen, insbesondere Fluor, Chlor oder Brom; Cyano, Nitro sowie Halogenalkyl mit 1 bis 2 Kohlenstoffatomen und 1 bis 3 gleichen oder verschiedenen Halogenatomen, wobei als Halogenatome vorzugsweise Fluor oder Chlor geeignet sind und als Beispiel für Halogenalkyl Trifluormethyl genannt wird.

$R^1$ steht bevorzugt für einen verzweigten oder unverzweigten Alkylrest mit 1 bis 10 Kohlenstoffatomen, beispielsweise Methyl, Ethyl, n-Propyl, i-Propyl, sec.-Butyl, neo-Pentyl, n-Hexyl, n-Heptyl, n-Octyl, n-Nonyl und n-Decyl. $R^1$ kann außerdem für Cycloalkylreste stehen wie z.B. Cyclopropyl, Cyclobutyl, Cyclopentyl, Cyclohexyl, Cycloheptyl, Cyclooctyl, wobei besonders Cyclopropyl, Cyclopentyl und Cyclohexyl genannt seien.

Ebenfalls vorteilhafte Verbindungen sind solche, in denen $R^1$ für Cycloalkyl-Alkylreste mit insgesamt 4 bis 11 Kohlenstoffatomen, wie z.B. die Cyclopropylmethyl-, Cyclobutylmethyl-, Cyclopentylmethyl-, Cyclohexylmethyl-, Cycloheptylmethyl-, Cyclopropylethyl-, Cyclobutylethyl-, Cyclopentylethyl- oder Cyclohexylethyl-Gruppe steht.

Außerdem kann $R^1$ einen ggf. substituierten Phenylrest oder einen ggf. subst. Phenylalkylrest bedeuten, wobei Substituenten Halogen (hier vorzugsweise Fluor oder Chlor), Cyano, Nitro, Alkyl, Alkyloxy, Alkylthioreste mit 1 bis 4 Kohlenstoffatomen im Alkylteil, Halogenalkylreste mit 1 bis 4 C-Atomen und 1 bis 9 Halogenatomen sein können. Als Beispiele seien die Benzyl-, die 4-Chlorbenzyl- und die 2,4-Dichlorbenzylgruppe genannt.

Weiterhin steht $R^1$ für einen ggf. substituierten Phenoxyalkylrest oder einen ggf. substituierten Benzyloxyalkylrest mit 2 bis 10 Kohlenstoffatomen in der Alkylkette und bis zu 5 gleichen oder verschiedenen Substituenten am Phenylring, die Halogen (hier vorzugsweise Fluor oder Chlor), Cyano, Nitro, Alkyl, Alkyloxy, Alkylthioreste mit 1 bis 4 Kohlenstoffatomen im Alkylteil sowie Halogenalkylreste mit 1 bis 4 C-Atomen und 1 bis 9 Halogenatomen sein können.

Als Beispiele seien Phenoxyethyl, Phenoxypropyl, Phenoxybutyl, Phenoxypentyl, Phenoxyhexyl, Phenoxyheptyl, Phenoxyoctyl, Phenoxynonyl, Phenoxydecyl, p-Chlorphenoxyethyl, p-Chlorphenoxypropyl, p-Chlorphenoxybutyl, 2,4-Dichlorphenoxyethyl, 2,4-Dichlorphenoxypropyl, 2,4-Dichlorphenoxybutyl, Benzyloxyethyl, Benzyloxypropyl, Benzyloxybutyl, Benzyloxypentyl, Benzyloxyhexyl, Benzyloxyheptyl, Benzyloxyoctyl, Benzyloxynonyl und Benzyloxydecyl genannt.

$R^2$ und $R^3$ stehen in Formel I unabhängig voneinander für niedere Alkylreste, wobei als Beispiele $CH_3$, $C_2H_5$ und $n\text{-}C_3H_7$ genannt werden. $R^2$ und $R^3$ stehen außerdem gemeinsam für ein Diradikal der Formel $-(CH_2)_m-$, wobei m für eine ganze Zahl von 2 bis 7, insbesondere 2 bis 6 steht.

Az steht in der Formel I für einen Azolylreste. Bevorzugt ist der 1(1,2,4-Triazolyl)-Rest, der 1-Imidazolyl-Rest und der 1-Pyrazolyl-Rest.

Y steht in Formel I für die C=O-Gruppe sowie für das Strukturelement
$-CR^4-$ wobei $R^4$ beispielsweise Wasserstoff, Methyl, Ethyl, Propyl,
$OR^5$
Butyl, Vinyl oder Allyl sein kann und als Beispiele für $R^5$ Wasserstoff, Methyl, Ethyl, Propyl, Allyl, Benzyl, 4-Chlorbenzyl und 2,4-Dichlorbenzyl genannt werden.

Als biologisch annehmbare Salze der Azolverbindungen seien beispielsweise die Sulfate, Hydrogensulfate, Nitrate, Hydrochloride, Hydrobromide, p-Toluolsulfonate und Oxalate genannt.

Erfindungsgemäße Metallkomplexe sind beispielsweise die Addukte von Zink oder Kupfer an die Verbindungen der Formel I, z.B. I x 1/2 $CuCl_2$ oder I x 1/2 $ZnCl_2$.

Die Verbindungen der Formel lassen sich herstellen, indem man einem der Schemata A - E folgt:

Schema A

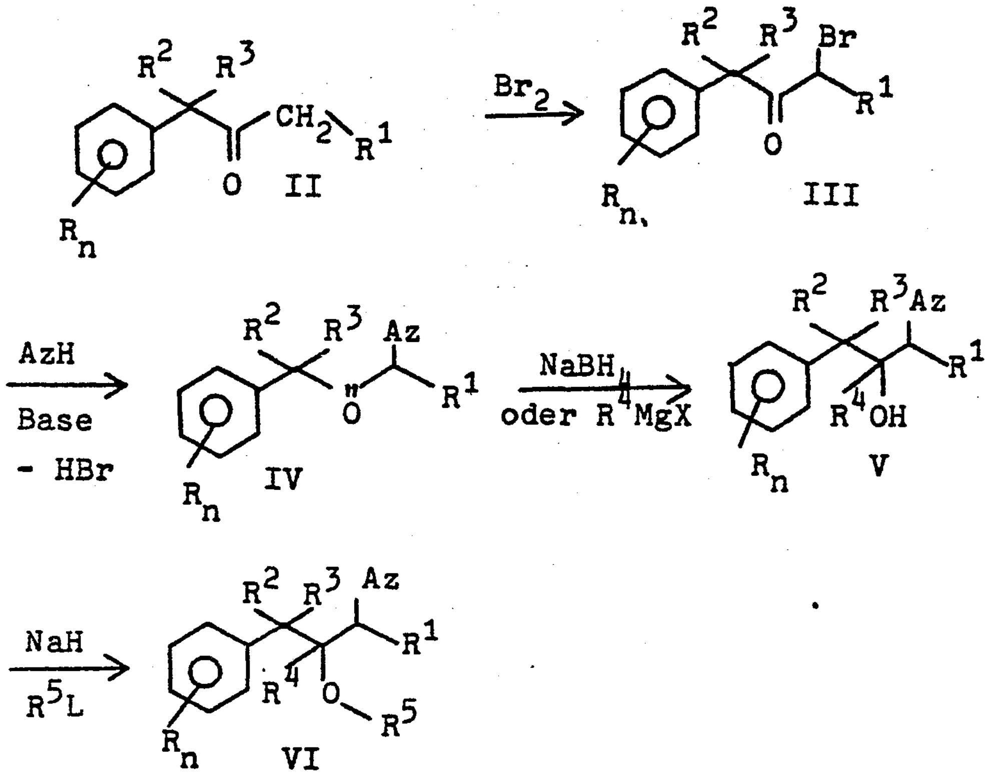

Schema B

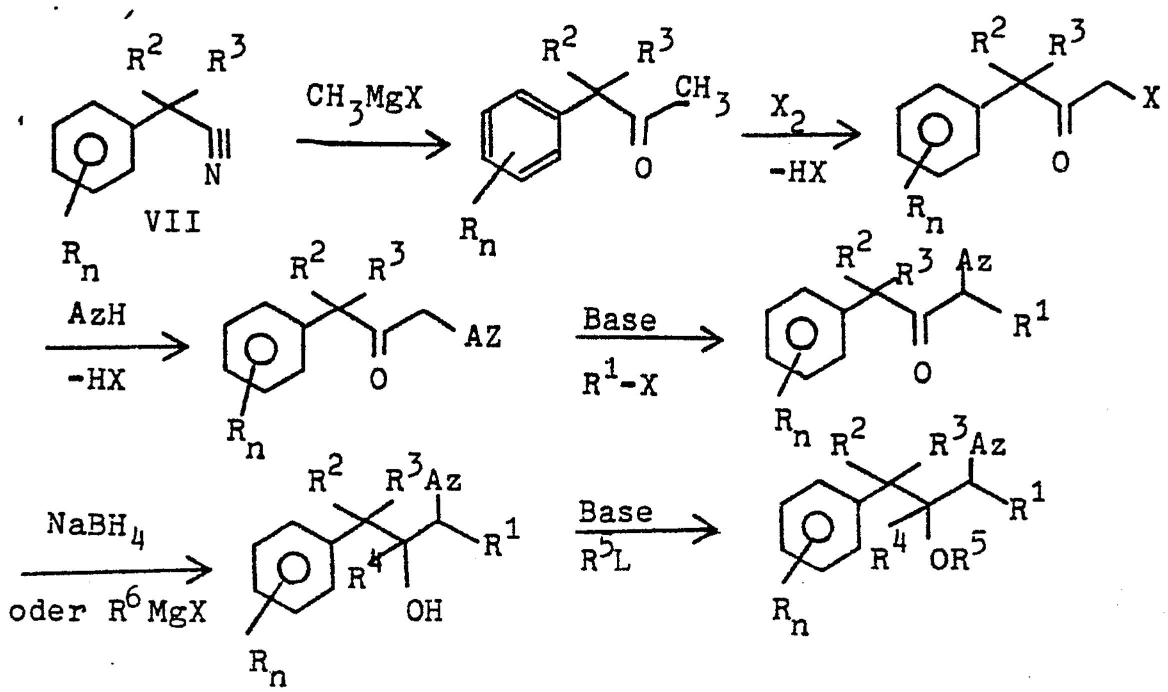


Schema C

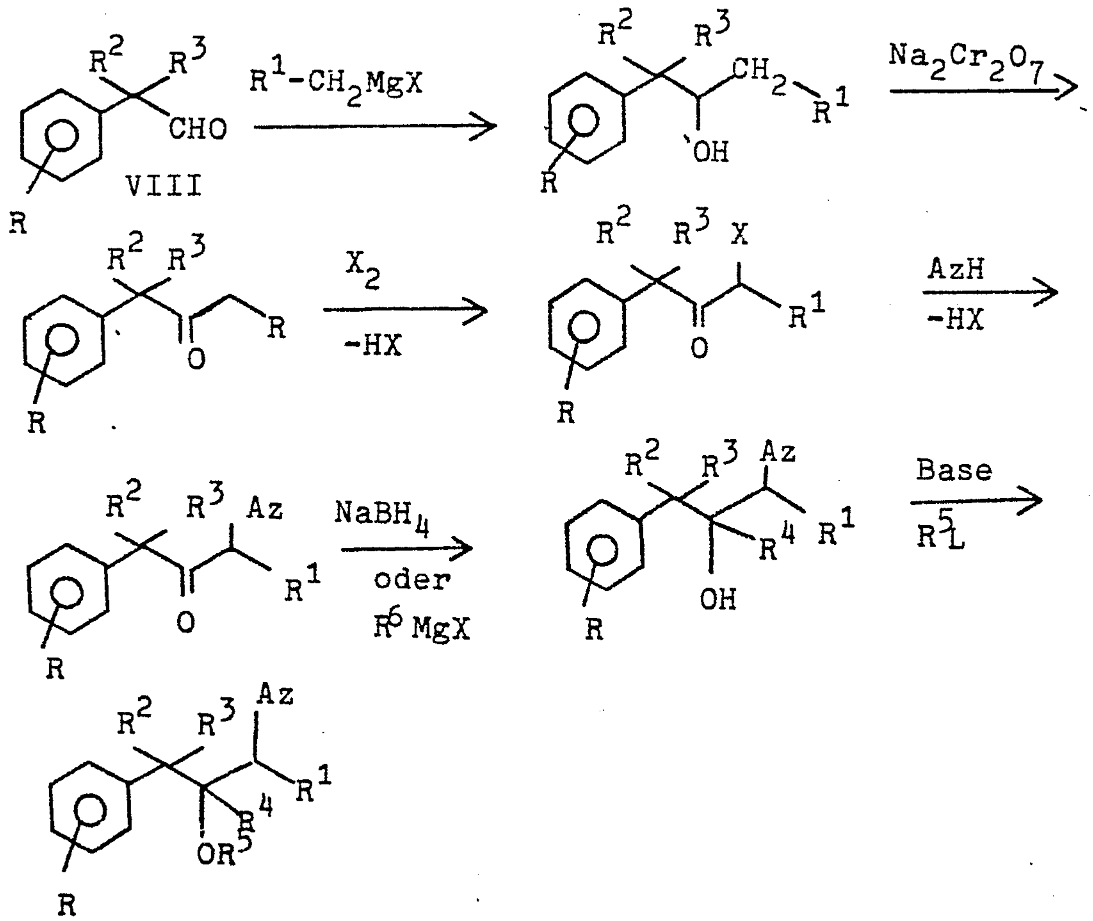

Schema D

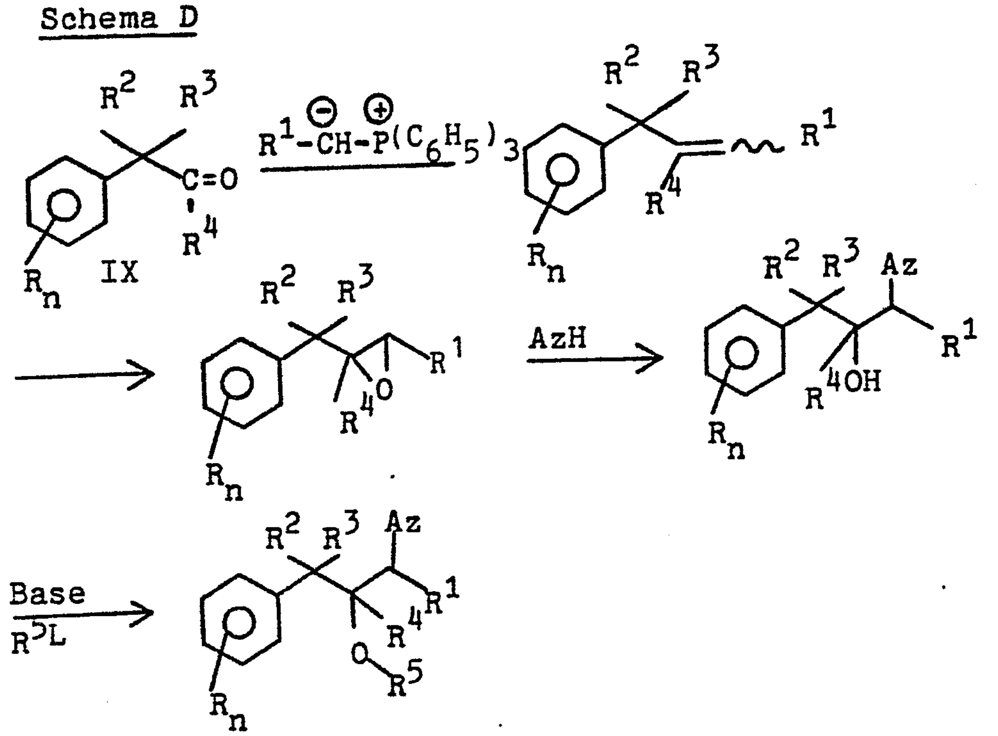


Schema E

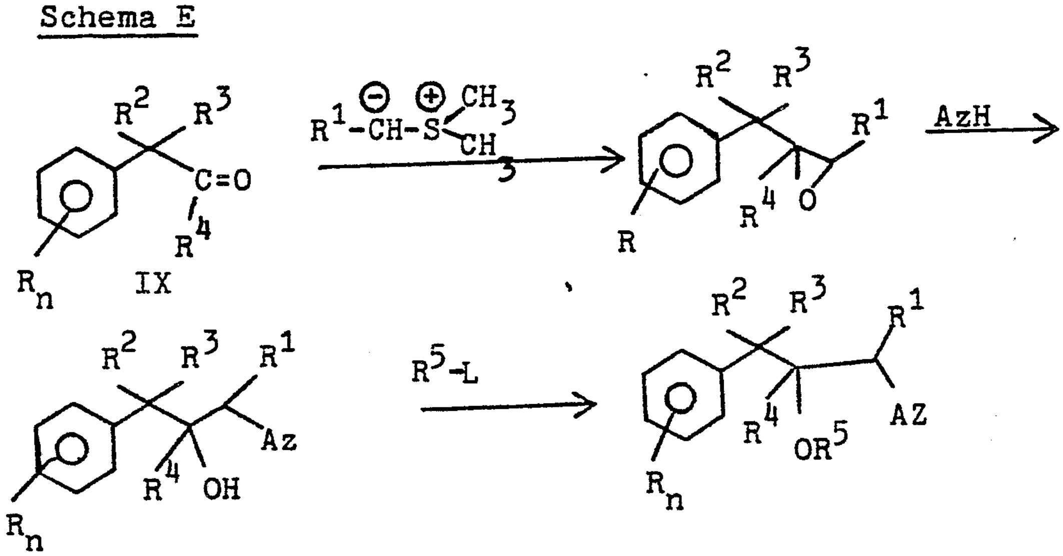


wobei R, $R^1$, $R^2$, $R^3$, $R^4$, $R^5$, Y, L, Az und n die oben angegebene Bedeutung haben und X Chlor, Brom und Iod bedeuten.

Die Ausgangsstoffe der Formel VII

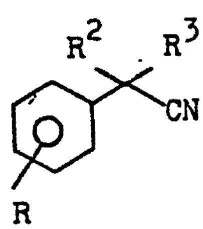

(VII)

sind bekannt oder können auf literaturbekannte Weise (vgl. z.B. Rosz. Chem. 39., 1595 (1965)) dargestellt werden.

Die Ausgangsverbindungen der Formeln II und IX sind bekannt oder lassen sich unter Verwendung bekannter Methoden aus Nitrilen der Formel VII herstellen.

Aldehyde der Formel VIII, die als Ausgangsmaterial in Schema C dienen, sind bekannt oder lassen sich in bekannter Weise durch Reduktion aus den Nitrilen der Formel VII darstellen.

Die Verbindungen der allgemeinen Formel I enthalten mindestens ein Chiralitätszentrum und können daher als Enantiomeren- bzw. Diastereomerengemische vorliegen. Die reinen Isomeren können entweder durch Auftrennung der Gemische oder durch gezielte Synthesen aus optisch reinen Ausgangsverbindungen sowie aus achiralen Vorläufern unter Verwendung chiraler Katalysatoren hergestellt werden.

Die einzelnen Isomeren haben im allgemeinen unterschiedliche biologische Aktivität und sind als solche ebenso wie ihre Gemische Gegenstand der vorliegenden Erfindung.

Die erfindungsgemäßen Komplexe und Säureadditionssalze sind durch Umsetzung von Verbindungen der allgemeinen Formel I mit Metallsalzen bzw. Säuren erhältlich. Sie sind meist kristalline Verbindungen mit guter Pflanzenverträglichkeit.

<u>Herstellbeispiel</u>

1a)

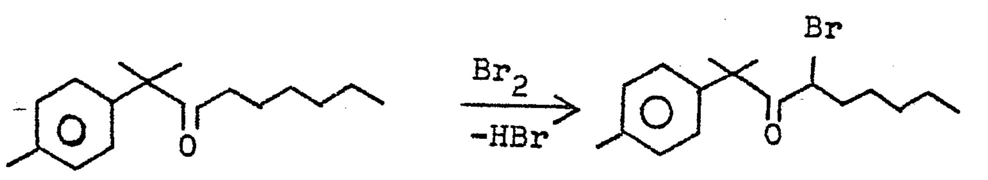


26,8 g (110 mmol) 2-Methyl-2-(4-Methylphenyl)-nonan-3-on werden in 50 ml Diethylether gelöst. Nach Zugabe von 0,1 g Aluminiumtrichlorid tropft man eine Lösung von 17,6 g (110 mmol) Brom in Diethylether zu und rührt bis zur weitgehenden Entfärbung des Reaktionsgemisches. Nach dem Waschen der organischen Phase mit 50 ml Eiswasser, Trocknen und Abdampfen des Lösungsmittels unter vermindertem Druck wird der Rückstand destilliert. Man erhält 20,6 g (58 % d. Th.) 4-Brom-2-methyl-2-(4-methylphenyl)-nonan-3-on, Kp = 150°C/0,5 mbar.

1b)

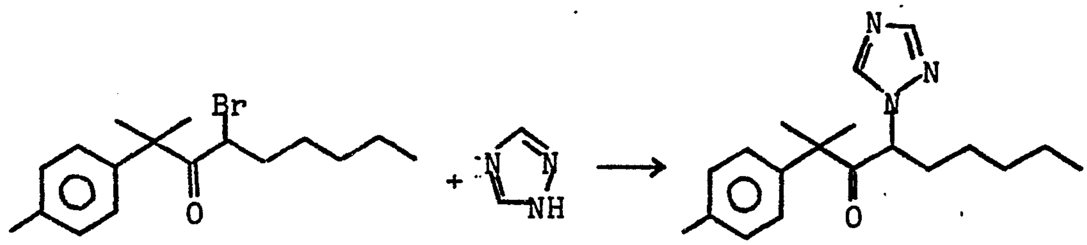

28 g (90 mmol) 4-Brom-2-methyl-2-(4-methylphenyl)-nonan-3-on und 12,2 g (180 mmol) 1,2,4-Triazol in 110 ml Aceton werden mit 20,7 g (150 mmol) $K_2CO_3$ 15 Stunden bei Raumtemperatur gerührt. Das Reaktionsgemisch wird vom Feststoff abgesaugt und das Lösungsmittel unter vermindertem Druck verdampft. Der Rückstand wird in Methylenchlorid aufgenommen, die organischen Phasen werden mit Wasser gewaschen und über Natriumsulfat getrocknet. Nach Kugelrohrdestillation erhält man 23,0 g (73 % d. Th.) 2-Methyl-2-(4-methylphenyl)-4-[1-(1,2,4-triazolyl)]-nonan-3-on als gelbliches Öl, Kp = 140°C/0,4 mbar.

2)

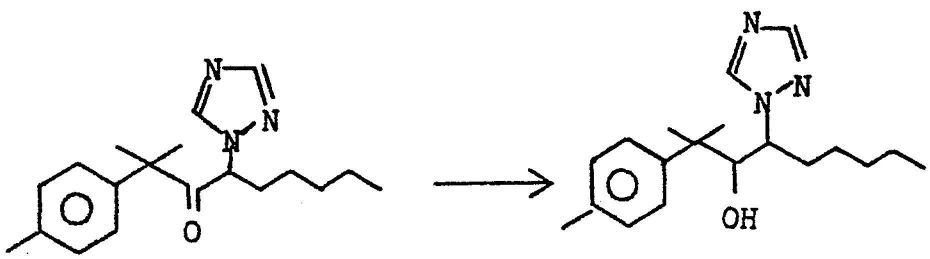

11 g (35 mMol) 2-Methyl-2-(4-methylphenyl)-4-[1-(1,2,4-triazolyl)]-nonan-3-on werden in 100 ml Isopropanol gelöst. Unter Kühlung auf 5 bis 10°C fügt man portionsweise 0,53 g (140 mMol) Natriumborhydrid zu und rührt 15 Stunden bei Raumtemperatur nach. Man zersetzt das Reaktionsgmisch mit verdünnter Schwefelsäure, konzentriert unter vermindertem Druck, gibt den Rückstand auf Eiswasser und extrahiert die wäßrige Phase mit Methylenchlorid. Nach dem Trocknen und Einengen des Lösungsmittels verbleibt ein Rückstand, der unter vermindertem Druck destilliert wird.

Man erhält 8,5 g (69,5 % d. Th.) 3-Hydroxy-2-(4-methylphenyl)-2-methyl-4-[1-(1,2,4-triazolyl)]-nonan, Kp = 155 - 160°C/0,4 mbar.

3)

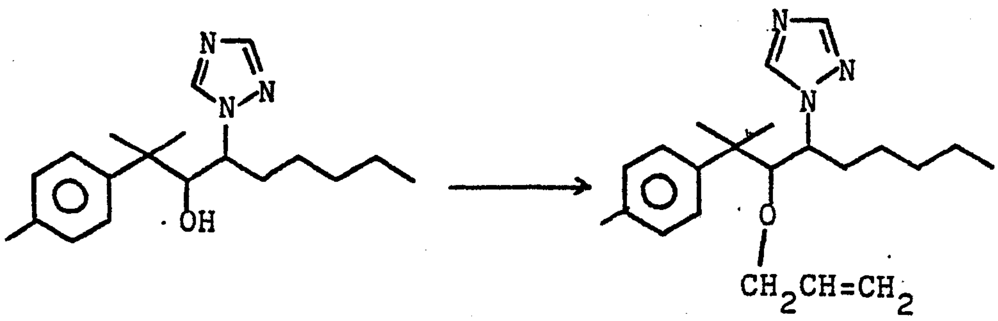

1,0 g (3,2 mMol) 3-Hydroxy-2-(4-methylphenyl)-2-methyl-4-[1-(1,2,4-triazolyl)]-nonan in 10 ml trockenem Diethylether werden mit 85 mg (3,5 mMol) Natriumhydrid versetzt und 10 Min. bei Raumtemperatur in einer Stickstoffatmosphäre gerührt. Man gibt 1 g (8,3 mMol) Allylbromid zu und kocht 1 Stunde am Rückfluß. Nach Zugabe von Wasser wird dreimal mit Ether extrahiert, über Natriumsulfat getrocknet und das Lösungsmittel abdestilliert. Der Rückstand ergibt nach Kugelrohrdestillation 1,0 g (94,5 % d. Th.) 3-Allyloxy-2-(4-methylphenyl)-2-methyl-4-[1-(1,2,4-triazolyl)]-nonan, Kp 180 - 185°C/0,9 mbar.

Nach dem vorstehenden Herstellbeispiel können durch Wahl anderer Ausgangsstoffe und entsprechende Anpassung der Verfahrensbedingungen die nachstehend aufgelisteten Verbindungen erhalten werden.

Soweit sie hergestellt wurden, sind ihre charakteristischen Siedepunkte angegeben oder, wenn solche nicht anzugeben waren, der äußere Aggregatzustand (Öl; Harz).

Die nicht charakterisierten Stoffe lassen aufgrund ihrer Struktur eine ähnliche biologische Wirkung erwarten wie die untersuchten Stoffe.

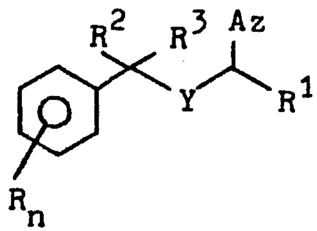

| Nr. | R | $R^1$ | $R^2$ | $R^3$ | Y | Az | Phys. Daten |
|---|---|---|---|---|---|---|---|
| 4 | 4-Cl | $CH_3$ | $CH_3$ | $CH_3$ | C=O | Imidazol | 200°C/0,5 mbar |
| 5 | 4-Cl | $CH_3$ | $CH_3$ | $CH_3$ | C=O | Triazol | 180°C/0,5 mbar |
| 6 | 4-Cl | $CH_3$ | $CH_3$ | $CH_3$ | HCOH | Triazol | 190°C/0,3 mbar |
| 7 | 4-Cl | $CH_3$ | $CH_3$ | $CH_3$ | HCOH | Imidazol | |
| 8 | 4-Cl | $C_2H_5$ | $CH_3$ | $CH_3$ | C=O | Imidazol | |
| 9 | 4-Cl | $C_2H_5$ | $CH_3$ | $CH_3$ | C=O | Triazol | |
| 10 | 4-Cl | $C_2H_5$ | $CH_3$ | $CH_3$ | HCOH | Imidazol | |
| 11 | 4-Cl | $C_2H_5$ | $CH_3$ | $CH_3$ | HCOH | Triazol | |
| 12 | 4-Cl | $n-C_3H_7$ | $CH_3$ | $CH_3$ | C=O | Triazol | 150°C/0,3 mbar |
| 13 | 4-Cl | $n-C_3H_7$ | $CH_3$ | $CH_3$ | HCOH | Triazol | 165 - 172°C/0,3 mbar |
| 14 | 4-Cl | $n-C_3H_7$ | $CH_3$ | $CH_3$ | HCO 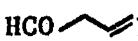 | Imidazol | Öl |
| 15 | 4-Cl | $n-C_3H_7$ | $CH_3$ | $CH_3$ | C=O | Imidazol | Öl |
| 16 | 4-Cl | $n-C_3H_7$ | $CH_3$ | $CH_3$ | HCOH | Imidazol | 250°C/1 mbar |
| 17 | 4-Cl | $n-C_4H_9$ | $CH_3$ | $CH_3$ | C=O | Imidazol | |

| Nr. | R | $R^1$ | $R^2$ | $R^3$ | Y | Az | Phys. Daten |
|---|---|---|---|---|---|---|---|
| 18 | 4-Cl | $n-C_4H_9$ | $CH_3$ | $CH_3$ | C=O | Triazol | |
| 19 | 4-Cl | $n-C_4H_9$ | $CH_3$ | $CH_3$ | HCOH | Imidazol | |
| 20 | 4-Cl | $n-C_4H_9$ | $CH_3$ | $CH_3$ | HCOH | Triazol | |
| 21 | 4-Cl | $-CH_2CH(CH_3)_2$ | $CH_3$ | $CH_3$ | C=O | Triazol/HCl | |
| 22 | H | $-CH_2CH(CH_3)_2$ | $CH_3$ | $CH_3$ | HCOH | Triazol | |
| 23 | 4-Cl | $-CH_2CH(CH_3)_2$ | $CH_3$ | $CH_3$ | C=O | Imidazol | |
| 24 | 4-Cl | $-CH_2CH(CH_3)_2$ | $CH_3$ | $CH_3$ | HCOH | Imidazol | |
| 25 | 4-Cl | $n-C_5H_{11}$ | $CH_3$ | $CH_3$ | C=O | Imidazol | |
| 26 | 4-Cl | $n-C_5H_{11}$ | $CH_3$ | $CH_3$ | C=O | Triazol | |
| 27 | 4-Cl | $n-C_5H_{11}$ | $CH_3$ | $CH_3$ | HCOH | Imidazol | 250°C/0,4 mbar |
| 28 | 4-Cl | $n-C_5H_{11}$ | $CH_3$ | $CH_3$ | HCOH | Triazol | |
| 29 | 4-Cl | $n-C_6H_{13}$ | $CH_3$ | $CH_3$ | C=O | Triazol | 200°C/0,5 mbar |
| 30 | 4-Cl | $n-C_6H_{13}$ | $CH_3$ | $CH_3$ | HCOH | Triazol | |
| 31 | 4-Cl | $-CH_2C(CH_3)_3$ | $CH_3$ | $CH_3$ | C=O | Triazol | 200°C/0,4 mbar |
| 32 | 4-Cl | $-CH_2C(CH_3)_3$ | $CH_3$ | $CH_3$ | HCOH | Triazol | |
| 33 | 4-Cl | 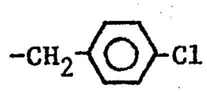 | $CH_3$ | $CH_3$ | C=O | Imidazol | |
| 34 | 4-Cl | 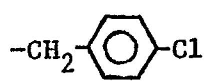 | $CH_3$ | $CH_3$ | C=O | Triazol | Harz |
| 35 | 4-Cl | 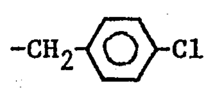 | $CH_3$ | $CH_3$ | HCOH | Imidazol | |
| 36 | 4-Cl | 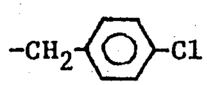 | $CH_3$ | $CH_3$ | HCOH | Triazol | Harz |

| Nr. | R | $R^1$ | $R^2$ | $R^3$ | Y | Az | Phys. Daten |
|---|---|---|---|---|---|---|---|
| 37 | 4-Cl | $-CH_2-$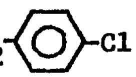$-Cl$ | $CH_3$ | $CH_3$ | C=O | Triazol | Fp. 126°C Zers. |
| 38 | 4-Cl | $n-C_8H_{17}$ | $CH_3$ | $CH_3$ | C=O | Imidazol | Öl |
| 39 | 4-Cl | $n-C_8H_{17}$ | $CH_3$ | $CH_3$ | C=O | Triazol | |
| 40 | 4-Cl | $n-C_8H_{17}$ | $CH_3$ | $CH_3$ | HCOH | Imidazol | |
| 41 | 4-Cl | $n-C_8H_{17}$ | $CH_3$ | $CH_3$ | HCOH | Triazol | |
| 42 | $4-OCH_3$ | $n-C_3H_7$ | $CH_3$ | $CH_3$ | C=O | Triazol | |
| 43 | $4-OCH_3$ | $n-C_3H_7$ | $CH_3$ | $CH_3$ | HCOH | Triazol | Kp 140°C/0,3 mbar |
| 44 | $3-CF_3$ | $n-C_3H_7$ | $CH_3$ | $CH_3$ | C=O | Triazol | Kp 140°C/0,2 mbar |
| 45 | $3-CF_3$ | $n-C_3H_7$ | $CH_3$ | $CH_3$ | HCOH | Triazol | Kp 130°C/0,4 mbar |
| 46 | 4-Br | $n-C_3H_7$ | $CH_3$ | $CH_3$ | C=O | Triazol | |
| 47 | 4-Br | $n-C_3H_7$ | $CH_3$ | $CH_3$ | HCOH | Triazol | Öl |
| 48 | 4-Br | $n-C_3H_7$ | $CH_3$ | $CH_3$ | HCO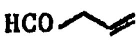 | Triazol | Öl |
| 49 | 4-Br | $n-C_3H_7$ | $CH_3$ | $CH_3$ | C=O | Imidazol | |
| 50 | 4-Br | $n-C_3H_7$ | $CH_3$ | $CH_3$ | HCOH | Imidazol | |
| 51 | $4-OCH_3$ | $n-C_3H_7$ | $CH_3$ | $CH_3$ | C=O | Imidazol | |
| 52 | $4-OCH_3$ | $n-C_3H_7$ | $CH_3$ | $CH_3$ | HCOH | Imidazol | |
| 53 | $4-OCH_3$ | $n-C_4H_7$ | $CH_3$ | $H_3$ | C=O | Triazol | |
| 54 | $4-OCH_3$ | $n-C_4H_9$ | $CH_3$ | $CH_3$ | HCOH | Triazol | |
| 55 | $4-OCH_3$ | $n-C_4H_9$ | $CH_3$ | $CH_3$ | C=O | Imidazol | |
| 56 | $4-OCH_3$ | $n-C_4H_9$ | $CH_3$ | $CH_3$ | HCOH | Imidazol | |
| 57 | $4-OCH_3$ | $n-C_3H_7$ | $-CH_2-CH_2-$ | | C=O | Imidazol | |
| 58 | $4-OCH_3$ | $n-C_3H_7$ | $-(CH_2)_3-$ | | C=O | Imidazol | |

| Nr. | R | $R^1$ | $R^2$ | $R^3$ | Y | Az | Phys. Daten |
|---|---|---|---|---|---|---|---|
| 59 | $4-OCH_3$ | $n-C_3H_7$ | | $-(CH_2)_4-$ | C=O | Imidazol | |
| 60 | $4-OCH_3$ | $n-C_3H_7$ | | $-(CH_2)_5-$ | C=O | Imidazol | |
| 61 | $4-OCH_3$ | $n-C_3H_7$ | | $-CH_2-CH_2-$ | HCOH | Imidazol | |
| 62 | $4-OCH_3$ | $n-C_3H_7$ | | $-(CH_2)_3-$ | HCOH | Imidazol | |
| 63 | $4-OCH_3$ | $n-C_3H_7$ | | $-(CH_2)_4-$ | HCOH | Imidazol | |
| 64 | $4-OCH_3$ | $n-C_3H_7$ | | $-(CH_2)_5-$ | HCOH | Imidazol | |
| 65 | $4-OCH_3$ | $n-C_3H_7$ | | $-CH_2-CH_2-$ | C=O | Triazol | |
| 66 | $4-OCH_3$ | $n-C_3H_7$ | | $-(CH_2)_3-$ | C=O | Triazol | |
| 67 | $4-OCH_3$ | $n-C_3H_7$ | | $-(CH_2)_4-$ | C=O | Triazol | |
| 68 | $4-OCH_3$ | $n-C_3H_7$ | | $-(CH_2)_5-$ | C=O | Triazol | |
| 69 | $4-OCH_3$ | $n-C_3H_7$ | | $-(CH_2)_2-$ | HCOH | Triazol | |
| 70 | $4-OCH_3$ | $n-C_3H_7$ | | $-(CH_2)_3-$ | HCOH | Triazol | |
| 71 | $4-OCH_3$ | $n-C_3H_7$ | | $-(CH_2)_4-$ | HCOH | Triazol | |
| 72 | $4-OCH_3$ | $n-C_3H_7$ | | $-(CH_2)_5$ | HCOH | Triazol | |
| 73 | H | $n-C_3H_7$ | | $-(CH_2)_2-$ | C=O | Imidazol | |
| 74 | H | $n-C_3H_7$ | | $-(CH_2)_3-$ | C=O | Imidazol | |
| 75 | H | $n-C_3H_7$ | | $-(CH_2)_4-$ | C=O | Imidazol | 200°C/0.4 mbar |
| 76 | H | $n-C_3H_7$ | | $-(CH_2)_5-$ | C=O | Imidazol | Öl |
| 77 | H | $n-C_3H_7$ | | $-(CH_2)_2-$ | HCOH | Imidazol | |
| 78 | H | $n-C_3H_7$ | | $-(CH_2)_3-$ | HCOH | Imidazol | |
| 79 | H | $n-C_3H_7$ | | $-(CH_2)_4-$ | HCOH | Imidazol | Öl |
| 80 | H | $n-C_3H_7$ | | $-(CH_2)_5-$ | HCOH | Imidazol | Öl |
| 81 | H | $n-C_3H_7$ | | $-(CH_2)_2-$ | C=O | Triazol | |

| Nr. | R | $R^1$ | $R^2$ | $R^3$ | Y | Az | Phys. Daten |
|---|---|---|---|---|---|---|---|
| 82 | H | $n-C_3H_7$ | | $-(CH_2)_3-$ | C=O | Triazol | |
| 83 | H | $n-C_3H_7$ | | $-(CH_2)_4-$ | C=O | Triazol | Öl |
| 84 | H | $n-C_3H_7$ | | $-(CH_2)_5-$ | C=O | Triazol | Öl |
| 85 | H | $n-C_3H_7$ | | $-(CH_2)_2-$ | HCOH | Triazol | |
| 86 | H | $n-C_3H_7$ | | $-(CH_2)_3-$ | HCOH | Triazol | |
| 87 | H | $n-C_3H_7$ | | $-(CH_2)_4-$ | HCOH | Triazol | Öl |
| 88 | H | $n-C_3H_7$ | | $-(CH_2)_5-$ | HCOH | Triazol | Öl |
| 89 | 4-Cl | $n-C_3H_7$ | $CH_3$ | $CH_3$ | $CH_3-C-OH$ | Triazol | |
| 90 | 4-Cl | $n-C_3H_7$ | $CH_3$ | $CH_3$ | 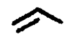 C-OH | Triazol | |
| 91 | 4-Cl | $n-C_3H_7$ | $CH_3$ | $CH_3$ | 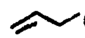C-OH | Triazol | |
| 92 | 4-Cl | $CH_2-CH(CH_3)_3$ | $CH_3$ | $CH_3$ | $CH_3-C-OH$ | Triazol | |
| 93 | 4-Cl | $CH_2-CH(CH_3)_3$ | $CH_3$ | $CH_3$ | 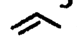 C-OH | Triazol | |
| 94 | 4-Cl | $CH_2-CH(CH_3)_3$ | $CH_3$ | $CH_3$ | 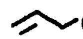C-OH | Triazol | |
| 95 | 4-Cl | $n-C_3H_7$ | | $-(CH_2)_2-$ | $CH_3-C-OH$ | Triazol | |
| 96 | 4-Cl | $n-C_3H_7$ | | $-(CH_2)_4-$ | $CH_3-C-OH$ | Triazol | |
| 97 | 4-Cl | $n-C_3H_7$ | | $-(CH_2)_5-$ | $CH_3-C-OH$ | Triazol | |
| 98 | 4-Cl | $n-C_3H_7$ | | $-(CH_2)_6-$ | $CH_3-C-OH$ | Triazol | |
| 99 | 4-Cl | $n-C_3H_7$ | $CH_3$ | $CH_3$ | $CH_3-C-OH$ | Imidazol | |
| 100 | 4-Cl | $n-C_3H_7$ | $CH_3$ | $CH_3$ | 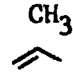 C-OH | Imidazol | |
| 101 | 4-Cl | $n-C_3H_7$ | $CH_3$ | $CH_3$ | 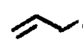C-OH | Imidazol | |
| 102 | 4-Cl | $n-C_3H_7$ | | $-(CH_2)_2-$ | $CH_3-C-OH$ | Imidazol | |
| 103 | 4-Cl | $n-C_3H_7$ | | $-(CH_2)-$ | $CH_3-C-OH$ | Imidazol | |

| Nr. | R | $R^1$ | $R^2$ | $R^3$ | Y | Az | Phys. Daten |
|---|---|---|---|---|---|---|---|
| 104 | 4-Cl | $n-C_3H_7$ | $-(CH_2)_5-$ | | $CH_3-C-OH$ | Imidazol | |
| 105 | 4-Cl | $-(CH_2)_2-O-$ 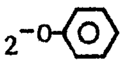 | $CH_3$ | $CH_3$ | C=O | Imidazol | |
| 106 | 4-Cl | $-(CH_2)_3-O-$ 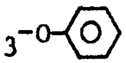 | $CH_3$ | $CH_3$ | C=O | Imidazol | |
| 107 | 4-Cl | $-(CH_2)_4-O-$ 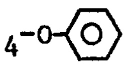 | $CH_3$ | $CH_3$ | C=O | Imidazol | |
| 108 | 4-Cl | $-(CH_2)_2-O-$ 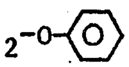 | $CH_3$ | $CH_3$ | C=O | Triazol | |
| 109 | 4-Cl | $-(CH_2)_3-O-$ 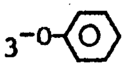 | $CH_3$ | $CH_3$ | C=O | Triazol | |
| 110 | 4-Cl | $-(CH_2)_4-O-$ 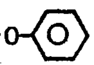 | $CH_3$ | $CH_3$ | C=O | Triazol | |
| 111 | 4-Cl | $-(CH_2)_2-O-$ 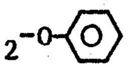 | $CH_3$ | $CH_3$ | HCOH | Triazol | |
| 112 | 4-Cl | $-(CH_2)_3-O-$ 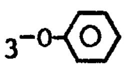 | $CH_3$ | $CH_3$ | HCOH | Triazol | |
| 113 | 4-Cl | $-(CH_2)_4-O-$ 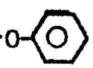 | $CH_3$ | $CH_3$ | HCOH | Triazol | |
| 114 | 4-Cl | $-(CH_2)_2-O-$ 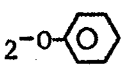 | $CH_3$ | $CH_3$ | HCOH | Imidazol | |

| Nr. | R | $R^1$ | $R^2$ | $R^3$ | Y | Az | Phys. Daten |
|---|---|---|---|---|---|---|---|
| 115 | 4-Cl | $-(CH_2)_3-O-$ 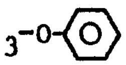 | $CH_3$ | $CH_3$ | HCOH | Imidazol | |
| 116 | 4-Cl | $-(CH_2)_4-O-$ 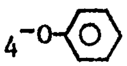 | $CH_3$ | $CH_3$ | HCOH | Imidazol | |
| 117 | 4-Cl | $-(CH_2)_2-O-CH_2-$ 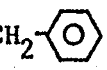 | $CH_3$ | $CH_3$ | C=O | Triazol | |
| 118 | 4-Cl | $-(CH_2)_2-O-CH_2-$ 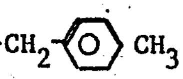 | $CH_3$ | $CH_3$ | HCOH | Triazol | |
| 119 | 4-Cl | $-(CH_2)_3-O-CH_2-$ 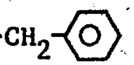 | $CH_3$ | $CH_3$ | C=O | Triazol | |
| 120 | 4-Cl | $-(CH_2)_3-O-CH_2-$ 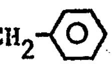 | $CH_3$ | $CH_3$ | HCOH | Triazol | |
| 121 | 4-Cl | $-(CH_3)_4-O-CH_2-$ 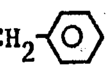 | $CH_3$ | $CH_3$ | C=O | Triazol | |
| 122 | 4-Cl | $-(CH_3)_4-O-CH_2-$ 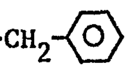 | $CH_3$ | $CH_3$ | HCOH | Triazol | |

Die neuen Verbindungen zeichnen sich durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Baumwolle, Soja, Kaffee, Zuckerrohr, Obst und Zierpflanzen im Gartenbau, Weinbau, sowie Gemüse - wie Gurken, Bohnen und Kürbisgewächse -.

Die neuen Verbindungen sind insbesondere geeignet zur Bekämpfung folgender Pflanzenkrankheiten:

Erysiphe graminis (echter Mehltau) in Getreide,
Erysiphe cichoracearum an Kürbisgewächsen,
Podosphaera leucotricha an Äpfeln,
Uncinula necator an Reben,
Puccinia-Arten an Getreide,
Rhizoctonia solani an Baumwolle,
Ustilago-Arten an Getreide und Zuckerrohr,
Venturia inaequalis (Schorf) an Äpfeln,
Septoria nodorum an Weizen,
Botrytis cinerea (Grauschimmel) an Erdbeeren, Reben,
Cercospora musae an Bananen,
Pseudocercosporella herpotrichoides an Weizen, Gerste,
Pyricularia oryzae an Reis,
Hemileia vastatrix an Kaffee,
Alternaria solani an Kartoffeln, Tomaten.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt z.B. prophylaktisch, d.h. vor der Infektion der Pflanzen oder Samen durch die Pilze; bereits befallene Pflanzen bzw. Kulturen können durch den Einsatz der erfindungsgemäßen Mittel häufig gerettet werden.

Die neuen Substanzen können in die üblichen Formulierungen übergeführt werden, wie Lösungen, Emulsionen, Suspensionen, Stäube, Pulver, Pasten und Granulate. Die Anwendungsform richtet sich nach dem vorgesehenen Verwendungszweck; sie soll in jedem Fall eine feine und gleichmäßige Verteilung der wirksamen Substanz gewährleisten. Die Formulierungen werden in

bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür z.B. in Frage: Lösungsmittel wie Aromaten (z.B. Xylol, Benzol), chlorierte Aromaten (z.B. Chlorbenzole), Paraffine (z.B. Erdölfraktionen), Alkohole (z.B. Methanol, Butanol), Amine (z.B. Ethanolamin, Dimethylformamid) und Wasser; Trägerstoffe wie natürliche Gesteinsmehle, z.B. Kaoline, Tonerden, Talkum, Kreide und synthetische Gesteinsmehle (z.B. hochdisperse Kieselsäure, Silikate); Emulgiermittel, wie nichtionogene und anionische Emulgatoren (z.B. Polyoxyethylen-Fettalkohol-Ether, Alkylsulfonate und Arylsulfonate) und Dispergiermittel, wie Lignin, Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmenge kann je nach den Umständen z.B. zwischen 0,02 und 3 kg Wirkstoff je ha betragen. Die neuen Verbindungen können auch zur Bekämpfung holzzerstörender Pilze wie Coniophora puteanea und Polystictus versicolor, z.B. als fungizid wirksame Bestandteile öliger Holzschutzmittel zum Schutz von Holz gegen holzverfärbende Pilze eingesetzt werden. Die Anwendung erfolgt in der Weise, daß man das Holz mit diesen Mitteln behandelt, beispielsweise tränkt oder anstreicht.

Die Mittel bzw. die daraus hergestellten gebrauchsfertigen Zubereitungen wie Lösungen, Emulsionen, Suspensionen, Pulver, Stäube, Pasten oder Granulate werden in bekannter Weise angewendet, beispielsweise durch Versprühen, Vernebeln, Verstäuben, Verstreuen, Beizen oder Gießen.

Beispiele für solche Zubereitungen sind:

I. Man vermischt 90 Gewichtsteile der Verbindung Nr. 1 mit 10 Gewichtsteilen N-Methyl-alpha-pyrrolidon und erhält eine Lösung, die zur Anwendung in Form kleinster Tropfen geeignet ist.

II. 20 Gewichtsteile der Verbindung Nr. 2 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol

Ethylenoxid an 1 Mol Ricinusöl besteht. Durch Ausgießen und feines Verteilen der Lösung in Wasser erhält man eine wäßrige Dispersion.

III. 20 Gewichtsteile der Verbindung Nr. 5 werden in einer Mischung gelöst, die aus 40 Gewichtsteilen Cyclohexanon, 30 Gewichtsteilen Isobutanol, 20 Gewichtsteilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

IV. 20 Gewichtsteile der Verbindung Nr. 6 werden in einer Mischung gelöst, die aus 25 Gewichtsteilen Cyclohexanol, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

V. 80 Gewichtsteile der Verbindung Nr. 12 werden den mit 3 Gewichtsteilen des Natriumsalzes der Diisobutylnaphthalin-alpha-sulfonsäure, 10 Gewichtsteilen des Natriumsalzes einer Ligninsulfonsäure aus einer Sulfitablauge und 7 Gewichtsteilen pulverförmigem Kieselsäuregel gut vermischt und in einer Hammermühle vermahlen. Durch feines Verteilen der Mischung in Wasser erhält man eine Spritzbrühe.

VI. 3 Gewichtsteile der Verbindung Nr. 13 werden mit 97 Gewichtsteilen feinteiligem Kaolin innig vermischt. Man erhält auf diese Weise ein Stäubemittel, das 3 Gew.% des Wirkstoffs enthält.

VII. 30 Gewichtsteile der Verbindung Nr. 1 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gewichtsteile der Verbindung Nr. 5 werden mit 10 Teilen Natriumsalz eines Phenolsulfonsäure-harnstoff-formaldehyd-Kondensates, 2 Teilen Kieselgel und 48 Teilen Wasser innig vermischt. Man erhält eine stabile wäßrige Dispersion. Durch Verdünnen mit Wasser erhält man eine wäßrige Dispersion.

IX. 20 Teile der Verbindung Nr. 6 werden mit 2 Teilen Calciumsalz der Dodecylbenzolsulfonsäure, 8 Teilen Fettalkoholpolyglykolether, 2 Teilen Natriumsalz eines Phenolsulfonsäure-harnstoff-formaldehyd--Kondensates und 68 Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung des Wirkungsspektrums.

Die folgende Liste von Fungiziden, mit denen die erfindungsgemäßen Verbindungen kombiniert werden können, soll die Kombinationsmöglichkeiten erläutern, nicht aber einschränken.

Fungizide, die mit den neuen Verbindungen kombiniert werden können, sind beispielsweise:

Schwefel,
Dithiocarbamate und deren Derivate, wie
Ferridimethyldithiocarbamat
Zinkdimethyldithiocarbamat
Zinkethylenbisdithiocarbamat
Manganethylenbisdithiocarbamat
Mangan-Zink-ethylendiamin-bis-dithiocarbamat
Tetramethylthiuramdisulfide
Ammoniak-Komplex von Zink-(N,N'-ethylen-bis-dithiocarbamat)
Ammoniak-Komplex von Zink-(N,N'-propylen-bis-dithiocarbamat)
Zink-(N,N'-propylen-bis-dithiocarbamat)
N,N'-Polypropylen-bis-(thiocarbamoyl)-disulfid

Nitroderivate, wie
Dinitro-(1-methylheptyl)-phenylcrotonat
2-sec.-Butyl-4,6-dinitrophenyl-3,3-dimethylacrylat
2-sec.-Butyl-4,6-dinitrophenyl-isopropylcarbonat
5-Nitro-isophthalsäure-di-isopropylester,

heterocyclische Strukturen, wie
2-Heptadecyl-2-imidazolin-acetat
2,4-Dichlor-6-(o-chloranilino)-s-triazin
0,0-Diethyl-phthalimidophosphonothioat
5-Amino-1-(bis-(dimethylamino)-phosphinyl)-3-phenyl-1,2,4- triazol)
2,3-Dicyano-1,4-dithiaanthrachinon
2-Thio-1,3-dithio-(4,5,6)-chinoxalin
1-(Butylcarbamoyl)-2-benzimidazol-carbaminsäuremethylester

2-Methoxycarbonylamino-benzimidazol
2-(Furyl-(2)-benzimidazol
2-(Thiazolyl-(4)-benzimidazol
N-(1,1,2,2-Tetrachlorethylthio)-tetrahydrophthalimid
N-Trichlormethylthio-tetrahydrophthalimid
N-Trichlormethyl-phthalimid

N-Dichlorfluormethylthio-N',N'-dimethyl-N-phenyl-schwefelsäurediamid
5-Ethoxy-3-trichlormethyl-1,2,3-thiadiazol
2-Rhodanmethylthiobenzthiazol
1,4-Dichlor-2,5-dimethoxybenzol
4-(2-Chlorphenylhydrazono)-3-methyl-5-isoxazolon
Pyridin-2-thio-1-oxid
8-Hydroxychinolin bzw. dessen Kupfersalz
2,3-Dihydro-5-carboxanilido-6-methyl-1,4-oxathiin
2,3-Dihydro-5-carboxanilido-6-methyl-1,4-oxathiin-4,4-dioxid
2-Methyl-5,6-dihydro-4-H-pyran-3-carbonsäure-anilid
2-Methyl-furan-3-carbonsäureanilid
2,5-Dimethyl-furan-3-carbonsäureanilid
2,4,5-Trimethyl-furan-3-carbonsäureanilid
2,5-Dimethyl-furan-3-carbonsäurecyclohexylamid
N-Cyclohexyl-N-methoxy-2,5-dimethyl-furan-3-carbonsäureamid
2-Methyl-benzoesäure-anilid
2-Iod-benzoesäure-anilid
N-Formyl-N-morpholin-2,2,2-trichlorethylacetal
Piperazin-1,4-diylbis-(1-(2,2,2-trichlor-ethyl)-formamid
1-(3,4-Dichloranilino)-1-formylamino-2,2,2-trichlorethan
2,6-Dimethyl-N-tridecyl-morpholin bzw. dessen Salze
2,6-Dimethyl-N-cyclododecyl-morpholin bzw. dessen Salze
N-[3-(p-tert.-Butylphenyl)-2-methylpropyl]-cis-2,6-dimethylmorpholin
N-[3-(p-tert.-Butylphenyl)-2-methylpropyl]-piperidin
1-[2-(2,4-Dichlorphenyl)-4-ethyl-1,3-dioxolan-2-yl-ethyl]-1H-1,2,4-triazol
1[2-(2,4-Dichlorphenyl)-4-n-propyl-1,3-dioxolan-2-yl-ethyl]-1-H-1,2,4-triazol
N-(n-Propyl)-N-(2,4,6-trichlorphenoxyethyl)-N'-imidazol-yl-harnstoff
1-(4-Chlorphenoxy)-3,3-dimethyl-1-(1H-1,2,4-triazol-1-yl)-2-butanon
1-(4-Chlorphenoxy)-3,3-dimethyl-1-(1H-1,2,4-triazol-1-yl)-2-butanol
α-(2-Chlorphenyl)-α-(4-chlorphenyl)-5-pyrimidin-methanol
5-Butyl-2-dimethylamino-4-hyroxy-6-methyl-pyrimidin
Bis-(p-chlorphenyl)-3-pyridinmethanol
1,2-Bis-(3-ethoxycarbonyl-2-thioureido)-benzol
1,2-Bis-(3-methoxycarbonyl-2-thioureido)-benzol

sowie verschiedene anderen Strukturgruppen zugerechnete Fungizide, wie
Dodecylguanidinacetat
3-(3-(3,5-Dimethyl-2-oxycyclohexyl)-2-hydroxyethyl)-gluataramid
Hexachlorbenzol
DL-Methyl-N-(2,6-dimethyl-phenyl)-N-furoyl(2)-alaninat,
DL-N-(2,6-Dimethyl-phenyl)-N-(2'-methoxyacetyl)-alaninmethylester
N-(2,6-Dimethylphenyl)-N-chloracetyl-D,L-2-aminobutyrolacton
DL-N-(2,6-Dimethylphenyl)-N-(phenylacetyl)-alaninmethylester
5-Methyl-5-vinyl-3-(3,5-dichlorphenyl)-2,4-dioxo-1,3-oxazolidin
3-(3,5-Dichlorphenyl(-5-methyl-5-methoxymethyl-1,3-oxazolidin-2,4-dion
3-(3,5-Dichlorphenyl)-1-isopropylcarbamoylhydantoin
N-(3,5-Dichlorphenyl)-1,2-dimethylcyclopropan-1,2-dicarbonsäureimid.

Anwendungsbeispiel 1

Wirksamkeit gegen Weizenmehltau

Blätter von in Töpfen gewachsenen Weizenkeimlingen der Sorte "Jubilar" werden mit wäßriger Spritzbrühe, die 80 % Wirkstoff und 20 % Emulgiermittel in der Trockensubstanz enthält, besprüht und 24 Stunden nach dem Antrocknen des Spritzbelages mit Oidien (Sporen) des Weizenmehltaus (Erysiphe graminis var. tritici) bestäubt. Die Versuchspflanzen werden anschließend im Gewächshaus bei Temperaturen zwischen 20 und 22°C und 75 bis 80 % relativer Luftfeuchtigkeit aufgestellt. Nach 7 Tagen wird das Ausmaß der Mehltauentwicklung ermittelt.

Bewertung:
0 = kein Pilzbefall, abgestuft bis
5 = Totalbefall

A = leichter Blattschaden
B = mittlerer Blattschaden
C = starker Blattschaden
T = Totalschaden

| Wirkstoff Nr. nach der Tab. | Befall der Blätter nach Applikation von .... %iger Wirkstoffbrühe 0,025 | 0,006 | 0,0015 |
|---|---|---|---|
| 1 | 1 | 2 | 2 |
| 2 | 0 | 1 | 0 |
| 6 | 0A | 0A | 3 |
| 13 | 0A | 1 | 3 |
| 22 | 0 | 0 | 1 |
| 31 | 0 | 0 | 3 |
| 36 | 1 | 1 | 2 |
| 43 | 0A | 0 | 1 |
| 45 | 0 | 0 | 2 |
| 47 | 0 | 1 | 2 |
| 48 | 0A | 0 | 1 |
| Vergleichsmittel bekannt aus EP 55 833 Wirkstoff Nr. 14 | 2 | 2 | 3-4 |
| Unbehandelt | | 4-5 | |

Anwendungsbeispiel 2

Wirksamkeit gegen Weizenbraunrost

Blätter von in Töpfen gewachsenen Weizensämlingen der Sorte "Jubilar" werden mit Sporen des Braunrostes (Puccinia recondita) bestäubt. Danach werden die Töpfe für 24 Stunden bei 20 bis 22°C in eine Kammer mit hoher Luftfeuchtigkeit (90 bis 95 %) gestellt. Während dieser Zeit keimen die Sporen aus und die Keimschläuche dringen in das Blattgewebe ein. Die infizierten Pflanzen werden anschließend mit wäßrigen Spritzbrühen, die 80 % Wirkstoff und 20 % Emulgiermittel in der Trockensubstanz enthalten, tropfnaß gespritzt. Nach dem Antrocknen des Spritzbelages werden die Versuchspflanzen im Gewächshaus bei Temperaturen zwischen 20 und 22°C und 65 bis 70 % relativer Luftfeuchte aufgestellt. Nach 8 Tagen wird das Ausmaß der Rostpilzentwicklung auf den Blättern ermittel.

Bewertung:

0 = kein Pilzbefall, abgestuft bis
5 = Totalbefall

A = leichter Blattschaden
B = mittlerer Blattschaden
C = starker Blattschaden
T = Totalschaden

| Wirkstoff Nr. nach der Tab. | Befall der Blätter nach Applikation von 0,025 %iger Wirkstoffbrühe |
|---|---|
| 13 | 2 |
| 22 | 1 |
| 28 | 2 |
| 36 | 1 |
| Vergleichsmittel bekannt aus EP 55 833 Wirkstoff Nr. 14 | 3-4A |
| Unbehandelt | 5 |

Anwendungsbeispiel 3

Wirksamkeit gegen Botrytis cinerea an Paprika

Paprikasämlinge der Sorte "Neusiedler Ideal Elite" werden, nachdem sich 4 bis 5 Blätter gut entwickelt haben, mit wäßrigen Suspensionen, die 80 % Wirkstoff und 20 % Emulgiermittel in der Trockensubstanz enthalten, tropfnaß gespritzt. Nach dem Antrocknen des Spritzbelages werden die Pflanzen mit einer Konidienaufschwemmung des Pilzes Botrytis cinerea besprüht und bei 22 bis 24°C in eine Kammer mit hoher Luftfeuchtigkeit gestellt. Nach 5 Tagen hat sich die Krankheit auf den unbehandelten Kontrollpflanzen so stark entwickelt, daß die entstandenen Blattnekrosen den überwiegenden Teil der Blätter bedecken.

Bewertung:
0 = kein Pilzbefall, abgestuft bis
5 = Totalbefall

| Wirkstoff Nr. | Befall der Blätter nach Applikation von 0,05 %iger Wirkstoffbrühe |
|---|---|
| 22 | 2 |
| 25 | 2 |
| 27 | 2 |
| Vergleichsmittel bekannt aus EP 55 833 Wirkstoff Nr. 14 | 5 |
| Unbehandelt | 5 |

## Patentansprüche

1. Azolverbindung der Formel I,

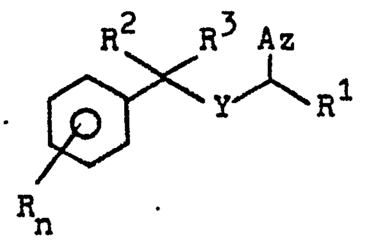

I,

in welcher

$R_n$ bis zu 5 gleiche oder verschiedene Substituenten bedeutet, ausgewählt aus der Gruppe Halogen, Alkyl, Alkoxy, Alkylthio, Alkylsulfonyl, Halogenalkyl, Nitro, Cyano, ggf. substituiertes Phenyl, ggf. substituiertes Phenoxy oder Wasserstoff,

$R^1$ einen ggf. verzweigten Alkylrest, einen Cycloalkylrest, einen Cycloalkylalkylrest, einen ggf. substituierten Arylrest, einen ggf. substituierten Aralkylrest, einen ggf. substituierten Aryloxyalkylrest, einen ggf. substituierten Benzyloxyalkylrest, einen ggf. substituierten Alkenylrest oder einen ggf. substituierten Alkinylrest bedeutet,

$R^2$ und $R^3$ unabhängig voneinander Alkyl oder gemeinsam ein Diradikal der Formel $-(CH_2)_m-$ bedeuten, wobei m für eine ganze Zahl von 2 bis 7 steht,

Az 1-(1,2,4-Triazolyl), 4-(1,2,4-Triazolyl), 1-Imidazolyl, 1-Pyrazolyl oder 1-Benzimidazolyl bedeutet,

Y 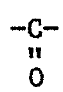 oder 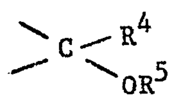 bedeutet, wobei

$R^4$ Wasserstoff, $C_1$- bis $C_4$-Alkyl, Vinyl oder Allyl und

$R^5$ Wasserstoff, $C_1$- bis $C_3$-Alkyl, ggf. subst. Alkenyl, ggf. subst. Alkinyl oder ggf. subst. Benzyl bedeutet.

2. Die biologisch annehmbaren Salze oder Komplexverbindungen der Verbindungen gemäß Anspruch 1.

3. Verfahren zur Herstellung von Azolverbindungen gemäß Anspruch 1 bzw. 2, in denen Y die -CO-Gruppe ist, dadurch gekennzeichnet, daß man ein entsprechendes α-Halogenketon der Formel II

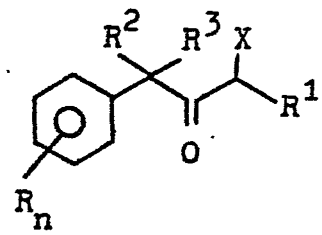 II,

in der X für Chlor, Brom oder Iod steht, bei einer ausreichenden Temperatur unterhalb von etwa 100°C mit einem entsprechenden Azol umsetzt.

4. Verfahren zur Herstellung von Azolverbindungen gemäß Formel I, in der Y die -CHOH-Gruppe bedeutet, dadurch gekennzeichnet, daß man eine Verbindung der Formel I, in der Y die CO-Gruppe bedeutet, bei einer ausreichenden Temperatur unterhalb von etwa 100°C entweder mit einem komplexen Hydrid oder mit Wasserstoff in Gegenwart eines Hydrierkatalysators behandelt.

5. Verfahren zur Herstellung von Azolverbindungen gemäß Formel I, in der Y die Gruppe 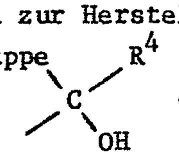 ($R^4$ von Wasserstoff verschieden) bedeutet, dadurch gekennzeichnet, daß man eine Verbindung der Formel I, in der Y die CO=Gruppe bedeutet, mit einer entsprechenden Grignardverbindung $R^4$-MgHal, in der Hal Chlor, Brom oder Iod bedeutet, gegebenenfalls in Gegenwart eines Magnesium- oder Tetraalkylammoniumhalogenids unterhalb von etwa 100°C umsetzt und das so entstandene Alkoholat zum tertiären Alkohol hydrolysiert.

6. Verfahren zur Herstellung von Verbindungen gemäß Formel I, in der Y die Gruppe 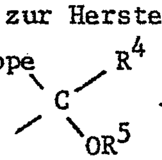 ($R^5$ von Wasserstoff verschieden) bedeutet, dadurch gekennzeichnet, daß man den nach Anspruch 5 erhaltenen tertiären Alkohol in das Alkalisalz oder das quartäre Ammoniumsalz überführt und dieses mit einem entsprechenden Alkylierungsmittel L-$R^5$, in der in der L für eine nucleophil verdrängbare Abgangsgruppe steht, unterhalb von 100°C umsetzt.

7. Pflanzenbehandlungsmittel, enthaltend eine Verbindung gemäß Anspruch 1 oder 2.

8. Verfahren zur Behandlung von Pflanzen, dadurch gekennzeichnet, daß man mindestens eine Verbindung gemäß Anspruch 1 oder 2 auf diese einwirken läßt.

9. Verfahren zur vorbeugenden Behandlung von Pflanzen, dadurch gekennzeichnet, daß man mindestens eine Verbindung gemäß Anspruch 1 oder 2 auf Pflanzen, deren Saatgut oder deren Anbaufläche einwirken läßt.

10. Verfahren zur Herstellung von Pflanzenbehandlungsmitteln, dadurch gekennzeichnet, daß man mindestens eine Verbindung gemäß Anspruch 1 oder 2 mit mindestens einem festen oder flüssigen Trägerstoff, oberflächenaktiven Mittel und ggf. weiteren, als Pflanzenbehandlungsmittel wirksamen Stoff mischt.

Europäisches Patentamt

0129798

Nummer der Anmeldung

# EUROPÄISCHER RECHERCHENBERICHT

EP 84106861.2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| X,D | EP - A1 - 0 055 833 (BAYER AG) * Patentansprüche * | 1,2,4, 7-10 | C 07 D 249/08<br>C 07 D 231/12<br>C 07 D 233/60<br>C 07 D 235/06<br>A 01 N 43/64<br>A 01 N 43/50<br>A 01 N 43/52<br>A 01 N 43/56 |
| A,D | DE - A1 - 3 048 266 (BAYER AG) * Patentansprüche * | 1-3,7-10 | |
| A | DE - A1 - 3 011 258 (BASF AG) * Patetnansprüche 1-3 * | 1,2,7-10 | |
| A | DE - A1 -3 140 276 (BAYER AG) * Patentansprüche 1,4-7 * | 1,2,7-10 | |
| A | DE - A1 - 3 003 933 (BASF AG) * Patentansprüche 1-3 * | 1,2,7-10 | |

RECHERCHIERTE SACHGEBIETE (Int. Cl. 3)

C 07 D 249/00
C 07 D 231/00
C 07 D 233/00
C 07 D 235/00

Der vorliegende Recherchenbericht wurde für alle Patentansprüche erstellt.

| Recherchenort | Abschlußdatum der Recherche | Prüfer |
|---|---|---|
| WIEN | 17-09-1984 | BRUS |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82